# EUROPEAN PATENT APPLICATION

(11) **EP 1 129 706 A2**
(43) Date of publication of application: **05.09.2001**
(21) Application number: 00309129.5
(22) Date of filing: 17.10.2000
(51) Int. Cl.: A61K 31/00, A61P 25/02

(54) **Use of cGMP PDE5 inhibitors for the treatment of neuropathy**

(30) Priority: 21.10.1999 GB 9924958; 01.09.2000 GB 0021520
(71) Applicant: Pfizer Limited, Sandwich Kent CT13 9NJ (GB); PFIZER INC., New York, N.Y. 10017 (US)
(72) Inventor: Grossman, Eric Ben, New York, New York 10017 (US); Koppiker, Nandan Parmanand, Sandwich, Kent CT13 9NJ (GB); Leichter, Steven B., Cataula, GA 31804-3603 (US)
(74) Representative: Hayles, James Richard

(57) **Abstract**

This invention relates to the use of cyclic guanosine 3', 5'-monophosphate phosphodiesterase type five (cGMP PDE5) inhibitors, including in particular the compound sildenafil, for the treatment of neuropathy, including in particular the treatment of diabetic neuropathy.

## Description

This invention relates to the use of cyclic guanosine 3', 5'-monophosphate phosphodiesterase type five (cGMP PDE5) inhibitors, including in particular the compound sildenafil, for the treatment of neuropathy, including in particular the treatment of diabetic neuropathy.

According to the specification of our International patent application WO94/28902 we have discovered that compounds which are inhibitors of the cGMP PDE5 enzyme are potent and effective compounds for the treatment of male erectile dysfunction (MED, impotence) and for female sexual disorders. This discovery led to the development of the compound sildenafil (5-[2-ethoxy-5-(4-methyl-1-piperazinylsulphonyl)phenyl]-1-methyl-3-n-propyl-1,6-dihydro-7H-pyrazolo[4,3-d]pyrimidin-7-one) (VIAGRA™) which has proved to be outstandingly successful as the first orally effective treatment for MED.

Neuropathy is a general term which describes a disease process which leads to the dysfunction of the nervous system. There are many causes of neuropathy affecting both the autonomic and peripheral nervous systems, such as metabolic disorders e.g. diabetes, hypothyroidism, porphyria; toxic substances e.g. alcohol and some heavy metals and drugs; infections and inflammatory conditions such as leprosy and the vasulitidis e.g. polyarteritis nodosa and systemic lupus as well as leukaemias, lymphomas and other paraneoplastic states. Neuropathy may also be associated with genetic or hereditary diseases as well as amyloidosis or dysproteinaemias.

In particular, neuropathy is caused by the following systemic diseases, such as diabetes mellitus (common), uremia (sometimes), porphyria, hypoglcemia, vitamin deficiency, vitamin B12 deficiency, critical illness (sepsis), chronic liver disease, primary billiary cirrhosis, primary systemic amyloidosis, hypothyroidism, chronic obstructive lung disease, acromegaly, malabsorption (sprue, celiac disease), carcinoma (sensory), carcinoma (sensorimotor), cancinoma (late), carcinoma (demyelinating), HIV infection, Lyme disease, lymphoma including Hodgkin's disease, polycythemia vera, multiple myeloma (lytic type), multiple myeloma (osteosclerotic or solitary plasmacytoma), benign monoclonal gammopathy (IgA, IgG and IgM) or cryoglobulinemia.

In addition, neuropathy is caused by drugs, such as amiodarone (antiarrhythmic), aurothioglucose (antirheumatic), cisplatin (antineoplastic), dapsone (dermatologic agent used e.g. for leprosy), disulfiram (antialcoholism agent), hydralazine (antihypertensive), isoniazid, metronidazle (antiprotozoal), misonidazole (radiosensitizer), nitofurantoin (urinary antiseptic), nucleoside analogues (ddC, ddl, d4T) (antiretroviral agents), phenytoin (anticonvulsant), pyridoxine (vitamin), suramin (antineoplastic), taxol (antineoplastic) or vincristine (antineoplastic).

In addition, neuropathy is caused by environmental toxins, such as acrylamide (flocculant/ grouting agent), arsenic (herbicide/ insecticide), diphtheria toxin, gamma-diketone hexacarbons, inorganic lead, organophosphates or thallium (rat poison).

Inaddition, neuropathy is caused by genetic disorders, such as Charcot-Marie-Tooth (CMT) disease (types 1A, 1B, 2 and 4A), hereditory amyloid polyneuropathies, Hereditory sensory neuropathy (types I and II), porphryivc neuropathy, hereditory liability to pressure palsy, Fabry's disease, adrenomyeloneuropathy, Dejerine-Sottas neuropathy (types A and B), Refsum's disease, ataxia-telangiectasia, Abetalipo-proteinemia, giant axonal neuropathy, metachromatic leukodystrophy, Frieddreich's ataxia.

Neuropathy is one of the major complications of diabetes mellitus, with no well-established therapies for either its symptomatic treatment or for prevention of progressive decline in nerve function. Estimates of the prevalence of polyneuropathy in diabetes vary widely (5% to 80%), largely due to the wide variety of definitions and clinical descriptions of polyneuropathy. Nevertheless, prevalence rates in the order of 20% have been recorded in both hospital and community-based studies in the UK.

Diabetic neuropathy is an umbrella term, itself encompassing a wide variety of clinical types of neuropathy which fall into two major groups of focal (mono-) and diffuse (poly-) neuropathies. Diabetic neuropathy is also a broad term that encompasses the peripheral, cranial and autonomic nerves that may be affected in diabetes mellitus. Subclassifications of diabetic neuropathy include a diffuse variety including such clinically distinct entities as distal symmetric sensory/sensorimotor (small/large/mixed fibre) polyneuropathy; autonomic neuropathy involving abnormalities in pupillary function, sweating, gastrointestinal (including gastric and gallbladder atony, diarrhoea), genito-urinary dysfunction (including bladder and sexual dysfunction) and cardiovascular autonomic neuropathy. Hypoglycaemic unawareness may be a manifestation of autonomic neuropathy as well. Focal diabetic neuropathies encompass mononeuropathies and mononeuropathy multiplex, the radiculopathies, the plexopathies, and cranial neuropathy. Chronic inflammatory demyelinating polyradiculoneuropathy may be focal or diffuse. Symmetrical polyneuropathies account for approximately 90% of clinical cases of diabetic polyneuropathy. Diabetic polyneuropathy includes in particular symmetrical sensorimotor polyneuropathy predominantly affecting the distal aspects of the lower limbs. Peripheral sensory neuropathies in diabetes may be acute or chronic in nature. Acute polyneuropathy often follows a sudden change in the metabolic state, is characterised by domination of 'positive' symptoms with few clinical signs, and usually resolves within 6-12 months. Chronic polyneuropathy has symptoms similar in nature to acute polyneuropathy, but has a more gradual onset without precipitating factors, usually has clinical signs, and may persist for many years.

Diabetic neuropathy may be classified according to the pattern of involvement of nerves into symmetrical or asymmetrical neuropathies. The former includes distal sensory and sensorimotor neuropathy, large-fibre type and small fibre types of neuropathy, distal small-fibre neuropathy, insulin neuropathy and chronic inflammatory demyelinating polyradiculoneuropathy (CIDP). Asymmetrical neuropathy due to diabetes includes mononeuropathy, mononeuropathy muliplex, radiculopathies, plexopathies and radiculoplexopathies and asymmetrical CIDP.

Clinically diabetic neuropathy may be classified as diffuse or focal.

The diffuse neuropathies comprise:
a) Distal symmetric sensorimotor polyneuropathy with subgroups of: i) primarily small-fibre manifesting as burning pain, cutaneous hyperaesthesia, paraesthesias, lancinating pain, loss of pain and temperature sensation, loss of visceral pain and foot ulceration; ii) primarily large-fibre manifesting as loss of vibration sensation, loss of proprioception, loss of reflexes and slowed nerve conduction velocities; and iii) mixed varieties; and
b) Autonomic neuropathy with subgroups of i) abnormal pupillary function; ii) sudomotor dysfunction (abnormalities of sweating); iii) genitourinary dysfunction, manifesting as bladder dysfunction and decreased bladder sensitivity/incontinence/retention; iv) sexual dysfunction including retrograde ejaculation, erectile dysfunction, defective lubrication (females); v) gastrointestinal dysfunction manifesting as gastroparesis, oesophageal motility disorders, gall bladder atony, diabetic diarrhoea or constipation; incontinence; or vi) hypoglycaemic unawareness (adrenal medullary neuropathy).
c) Cardiovascular dysfunction manifesting as resting tachycardia, impaired exercise-induced vardiovascular responses, cardiac denervation, heat intolerance, impaired vasodilatation, impaired venoarterial reflex dependent oedema, or orthostatic hypotension.
d) Hypoglycaemic unawareness.

The focal neuropathies comprise:
a) mononeuropathy
b) mononeuropathy multiplex
c) plexopathy
d) radiculopathy
e) cranial neuropathy
f) limb neuropathy including proximal diabetic neuropathy of lower limbs

The exact aetiopathogenesis of diabetic polyneuropathy remains unclear and is almost certainly multifactorial, with genetic predisposition, metabolic and vascular abnormalities, and lack or perturbation of growth factors, implicated. The response of the peripheral nervous system to the metabolic insults received in diabetes does not seem to differ between type 1 and type 2 diabetes, suggesting the likelihood of a similar clinical response to therapies in the two primary forms of the disease.

To date, none of the drugs tested for diabetic polyneuropathy convincingly alleviates symptoms, and none can lead to recovery of advanced, established polyneuropathy characterised by major axon loss. New, more effective therapies, are required.

WO99/54333 (unpublished at the priority date) and unpublished UK applications GB9924041.8 and GB9924063 describe substituted 5-(3-pyridyl)pyrazolo[4,3-d]pyrimidin-7-ones for treating peripheral diabetic neuropathy. Unpublished UK applications GB-A-9924028.5 and GB0007345.2 describe substituted 2-(3-pyridyl)-4a,5-dihydroimidazo[5,1-*f*][1,2,4]triazin-4(3*H*)-ones for treating peripheral diabetic neuropathy. Unpublished UK application GB9924020.2 describes substituted 2-phenylpurin-6-ones or substituted 2-(3-pyridyl)purin-6-ones for treating peripheral diabetic neuropathy. These compounds are disclaimed from the invention for treating peripheral diabetic neuropathy.

According to one aspect, the invention provides a method of treating a patient suffering from neuropathy (preferably diabetic polyneuropathy) which comprises treating said patient with an effective amount of a cGMP PDE5 inhibitor, with the proviso that the inhibitor is not a:
i) substituted 5-(3-pyridyl)pyrazolo[4,3-d]pyrimidin-7-one,
ii) substituted 2-(3-pyridyl)-4a,5-dihydroimidazo[5,1-*f*][1,2,4]triazin-4(3*H*)-one, or
iii) substituted 2-phenylpurin-6-one or a substituted 2-(3-pyridyl)purin-6-one, for treating peripheral diabetic neuropathy.

According to a second aspect, the invention provides the use of a cGMP-PDE5 inhibitor for the manufacture of a medicament for the treatment of neuropathy (preferably diabetic polyneuropathy),with the proviso that the inhibitor is not a:
i) substituted 5-(3-pyridyl)pyrazolo[4,3-d]pyrimidin-7-one,
ii) substituted 2-(3-pyridyl)-4a,5-dihydroimidazo[5,1-*f*][1,2,4]triazin-4(3*H*)-one, or
iii) substituted 2-phenylpurin-6-ones or a substituted 2-(3-pyridyl)purin-6-ones, for treating peripheral diabetic neuropathy.

The term neuropathy includes all classification of neuropathy described hereinabove. In addition the term neuropathy is not restricted to a particular cause, but includes all causes, in particular those described hereinabove.

Suitable cGMP PDE5 inhibitors for the use according to the present invention include:
the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124.

It is to be understood that the contents of the above published patent applications, and in particular the general formulae and exemplified compounds therein are incorporated herein in their entirety by reference thereto.

Preferred type V phosphodiesterase inhibitors for the use according to the present invention include:
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see Example 1 hereinafter);
5-[2-iso-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see Example 2 hereinafter);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see Example 3 hereinafter);
5-(5-Acetyl-2-propoxy-3-pyridinyl)-3-ethyl-2-(1-isopropyl-3-azetidinyl)-2,6-dihydro-7H-pyrazolo[4,3-*d*]pyrimidin-7-one (see Example 4 hereinafter);
5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (see Example 5 hereinafter);
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (IC-351), i.e. the compound of examples 78 and 95 of published international application WO95/19978, as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e. the compound of examples 20, 19, 337 and 336 of published international application WO99/24433; and
the compound of example 11 of published international application WO93/07124 (EISAI); and
compounds 3 and 14 from Rotella D P, *J. Med. Chem*., 2000, 43, 1257.

Still other type cGMP PDE5 inhibitors useful in conjunction with the present invention include:4-bromo-5-(pyridylmethylamino)-6-[3-(4-chlorophenyl)-propoxy]-3(2H)pyridazinone; 1-[4-[(1,3-benzodioxol-5- ylmethyl)amiono]-6-chloro-2-quinozolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3- (2H)pyridazinone; I-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)arnino]-6-chloro-2-quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an IC50 at less than 100 nanomolar, more preferably, at less than 50 nanomolar, more preferably still at less than 10 nanomolar.

IC50 values for the cGMP PDE5 inhibitors may be determined using established literature methodology, for example as described in EP0463756-B1 and EP0526004-A1.

Preferably the cGMP PDE5 inhibitors used in the invention are selective for the PDE5 enzyme. Preferably they are selective over PDE3, more preferably over PDE3 and PDE4. Preferably, the cGMP PDE5 inhibitors of the invention have a selectivity ratio greater than 100 more preferably greater than 300, over PDE3 and more preferably over PDE3 and PDE4.

Selectivity ratios may readily be determined by the skilled person. IC50 values for the PDE3 and PDE4 enzyme may be determined using established literature methodology, see S A Ballard *et al,* Journal of Urology, 1998, vol. 159, pages 2164-2171.

Surprisingly, the cGMP PDE5 inhibitors, such as sildenafil, can be used to treat neuropathy systemically, preferably by mouth.

The cGMP PDE5 inhibitors can be administered alone but, in human therapy will generally be administered in admixture with a suitable pharmaceutical excipient diluent or carrier selected with regard to the intended route of administration and standard pharmaceutical practice.

For example, the cGMP PDE5 inhibitors can be administered orally, buccally or sublingually in the form of tablets, capsules, ovules, elixirs, solutions or suspensions, which may contain flavouring or colouring agents, for immediate-, delayed-, modified-, or controlled-release applications.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethyl cellulose, hydroxypropylcellulose, sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the cGMP PDE5 inhibitors of the invention may be combined with various sweetening or flavouring agents, colouring matter or dyes, with emulsifying and/or suspending agents and with diluents such as water, ethanol, propylene glycol and glycerin, and combinations thereof.

The cGMP PDE5 inhibitors can also be administered parenterally, for example, intravenously, intra-arterially, intraperitoneally, intramuscularly or subcutaneously, or they may be administered by infusion techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The dosage of cGMP PDE5 inhibitor in such formulations will depend on its potency, but can be expected to be in the range of from 1 to 500 mg for administration up to three times a day. For oral and parenteral administration to human patients, the daily dosage level of the cGMP PDE5 inhibitor will usually be from 5 to 500 mg (in single or divided doses). In the case of sildenafil, a preferred dose is in the range 10 to 100 mg which can be administered up to three times a day. However the precise dose will be as determined by the prescribing physician and will depend on the age and weight of the patient and severity of the symptoms.

Thus, for example, tablets or capsules of the cGMP PDE5 inhibitor may contain from 5 to 250 mg (e.g. 10 to 100 mg) of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The cGMP PDE5 inhibitors can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane or 1,1,1,2,3,3,3-heptafluoropropane, carbon dioxide or other suitable gas. In the case of a pressurised aerosol, the dosage unit may be determined by providing a valve to deliver a metered amount. The pressurised container, pump, spray or nebuliser may contain a solution or suspension of the cGMP PDE5 inhibitor, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the cGMP PDE5 inhibitor and a suitable powder base such as lactose or starch.

Aerosol or dry powder formulations are preferably arranged so that each metered dose or "puff" contains from 1 to 50 mg of the cGMP PDE5 inhibitor, for delivery to the patient. The overall daily dose with an aerosol will be in the range of from 1 to 50 mg which may be administered in a single dose or, more usually, in divided doses throughout the day.

Alternatively, the cGMP PDE5 inhibitors can be administered in the form of a suppository or pessary.

The cGMP PDE5 inhibitor may be applied topically in the form of a gel, hydrogel, lotion, solution, cream, ointment or dusting powder. The cGMP PDE5 inhibitors may also be dermally or transdermally administered, for example, by the use of a skin patch.

For application topically to the skin, the cGMP PDE5 inhibitors can be formulated as a suitable ointment containing the inhibitor suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The cGMP PDE5 inhibitors may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

Generally, in humans, oral administration of the cGMP PDE5 inhibitors is the preferred route, being the most convenient. In circumstances where the recipient suffers from a swallowing disorder or from impairment of drug absorption after oral administration, the drug may be administered parenterally, sublingually or buccally.

The cGMP PDE5 inhibitors can also be administered in combination with other active agents. Preferred agents include: compounds which modulate the action of atrial natriuretic factor (also known as atrial natriuretic peptide), such as inhibitors of neutral endopeptidase; compounds which inhibit angiotensin-converting enzyme such as enalapril, and combined inhibitors of angiotensin-converting enzyme and neutral endopeptidase such as omapatrilat; angiotensin receptor antagonists such as losartan; substrates for NO-synthase, i.e. L-arginine; calcium-channel blockers such as amlodipine; antagonists of endothelin receptors and inhibitors of endothelin-converting enzyme; cholesterol lowering agents e.g. statins and fibrates; antiplatelet and antithrombotic agents, e.g. tPA, uPA, warfarin, hirudin and other thrombin inhibitors, heparin, thromboplastin activating factor inhibitors; insulin sensitising agents such as rezulin and hypoglycaemic agents such as glipizide; L-DOPA and carbidopa; acetylcholinesterase inhibitors such as donezipil or steroidal; COX2 inhibitors; pregabalene; gabapentene; tricyclic antidepressants, e.g. amitriptiline; non-steroidal anti-inflammatory agents; and angiotensin-converting enzyme (ACE) inhibitors, e.g. quinapril. More preferred agents are: compounds which inhibit angiotensin-converting enzyme; angiotensin receptor antagonists; substrates for NO-synthase antagonists of endothelin receptors and inhibitors of endothelin-converting enzyme; cholesterol lowering agents; and insulin sensitising agents and hypoglycaemic agents. Especially, insulin sensitising agents and hypoglycaemic agents.

It is to be appreciated that all references herein to treatment include curative, palliative and prophylactic treatment.

The following formulation examples are illustrative only and are not intended to limit the scope of the invention . Active ingredient means a cGMP PDE5 inhibitor.

### Formulation 1:

A tablet is prepared using the following ingredients :
Sildenafil citrate (50 mg) is blended with cellulose (microcrystalline), silicon dioxide, stearic acid (fumed) and the mixture is compressed to form tablets.

### Formulation 2 :

An intravenous formulation may be prepared by combining active ingredient (100 mg) with isotonic saline (1000 ml)

The efficacy of the cGMP PDE5 inhibitors in treating neuropathy in human patients was demonstrated by the following clinical trial.

The study was conducted using sidenafil, however it will be appreciated that the study may be conducted with other cGMP PDE5 inhibitors, for example one or more of the preferred cGMP PDE5 inhibitors listed hereinabove.

A number of men demonstrating positive symptoms of diabetic neuropathy were selected. The patients then underwent a 7-day treatment-free phase in order to establish baseline data, including a series of pain assessments comprising determining the Pain Disability Index (PDI) (adapted from Tait *et al 1990),* the Visual Analogue Scale (VAS) Pain Score, and the Verbal Evaluation of Pain Relief. They were then treated with either sildenafil (50 mg) or placebo every night for 10 days and immediately after this period the patient's degree of pain was reassessed. A washout period of 10 days followed where the patients received no treatment. Each patient was then treated with the alternative treatment (i.e. if they had originally taken active agent they were given placebo and *vice* versa) every night for a further 10 days. Immediately after this period the patient's degree of pain was assessed.

Pain Disability Index (PDI) is determined by patient questionnaire, and measures the overall impact of pain (on a scale of 0 to 10) associated with normal daily activities, (i.e. family/home responsibilities, recreation, social activity, occupation, self-care, sleeping). Since the pain of diabetic neuropathy is often at a maximum at night the PDI assessment was modified to reflect this.

Visual Analogue Scale (VAS) Pain Score is determined by the patient indicating on a continuous line from 0 (=no pain) to 100 (= pain as bad as it could be) the average level of a pain experienced during the assessment period. The pain is split into three catagories: superficial pain (burning sensations, tingling etc), deep pain (pins and needles, electric-like pain, numbing pain) and Muscular pain (deep aches, toothache-like pain, spasms, cramps).

The Verbal Evaluation of Pain Relief assessment was completed by the patient to indicate, on a scale of 0 to 6, the average amount of pain relief he has experienced over the past 10 days relative to baseline for that treatment period.

The results demonstrated a reduction in the degree of pain experienced in a number of patients, confirming the utility of cGMP PDE5 inhibitors for this indication.

Furthermore, anecdotal reports of improvement in painful symptoms in patients with diabetic polyneuropathy following single doses of 50 mg of sildenafil have been received.

### Example 1

### 2-(Methoxyethyl)-5-[2-ethoxy-5-(4-ethylpiperazin-1-ylsulphonyl)pyridin-3-yl]-3-ethyl-2,6-dihydro-7H-pyrazolo[4,3-d]pyrimidin-7-one

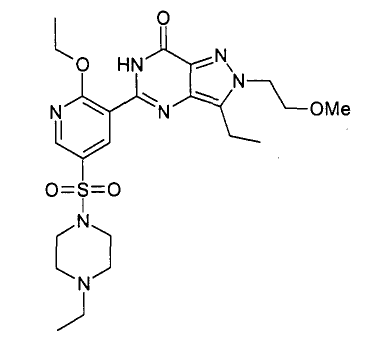

A mixture of the product from stage i) below (0.75mmol), potassium bis(trimethylsilyl)amide (298mg, 1.50mmol) and ethyl acetate (73 microlitres, 0.75mmol) in ethanol (10ml) was heated at 120°C in a sealed vessel for 12 hours. The cooled mixture was partitioned between ethyl acetate and aqueous sodium bicarbonate solution, and the layers separated. The organic phase was dried (MgSO₄), and evaporated under reduced pressure. The crude product was purified by column chromatography on silica gel using dichloromethane:methanol (98:2) as eluant to afford the title compound, 164mg; Found : C, 53.18; H, 6.48; N, 18.14; C₂₃H₃₃N₇O₅S;0.20C₂H₅CO₂CH₃ requires C, 53.21; H, 6.49; N, 18.25%; δ (CDCl₃) : 1.04 (3H, t), 1.40 (3H, t), 1.58 (3H, t), 2.41 (2H, q), 2.57 (4H, m), 3.08 (2H, q), 3.14 (4H, m), 3.30 (3H, s), 3.92 (2H, t), 4.46 (2H, t), 4.75 (2H, q), 8.62 (1H, d), 9.04 (1H, d), 10.61 (1H, s); LRMS : m/z 520 (M+1)⁺; mp 161-162°C.

### Preparation of Starting Materials

a) Pyridine-2-amino-5-sulphonic acid 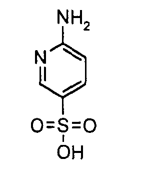 2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the title compound as a solid, 111.3g; LRMS : m/z 175 (M+1)⁺.
b) Pyridine-2-amino-3-bromo-5-sulphonic acid 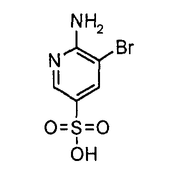 Bromine (99g, 0.62mol) was added dropwise over an hour, to a hot solution of the product from stage a) (108g, 0.62mol) in water (600ml) so as to maintain a steady reflux. Once the addition was complete the reaction was cooled and the resulting mixture filtered. The solid was washed with water and dried under suction to afford the title compound, 53.4g; δ (DMSOd₆, 300MHz): 8.08 (1H, s), 8.14 (1H, s); LRMS: m/z 253 (M)⁺.
c) Pyridine-3-bromo-2-chloro-5-sulphonyl chloride 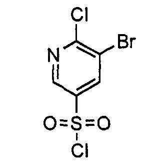 A solution of sodium nitrite (7.6g, 110.0mmol) in water (30ml) was added dropwise to an ice-cooled solution of the product from stage b) (25.3g, 100.0mmol) in aqueous hydrochloric acid (115ml, 20%), so as to maintain the temperature below 6°C. The reaction was stirred for 30 minutes at 0°C and for a further hour at room temperature. The reaction mixture was evaporated under reduced pressure and the residue dried under vacuum at 70°C for 72 hours. A mixture of this solid, phosphorus pentachloride (30.0g, 144mmol) and phosphorus oxychloride (1ml, 10.8mmol) was heated at 125°C for 3 hours, and then cooled. The reaction mixture was poured onto ice (100g) and the resulting solid filtered, and washed with water. The product was dissolved in dichloromethane, dried (MgSO₄), and evaporated under reduced pressure to afford the title compound as a yellow solid, 26.58g; δ (CDCl₃, 300MHz) : 8.46 (1H, s), 8.92 (1H, s).
d) 3-Bromo-2-chloro-5-(4-ethylpiperazin-1-ylsulphonyl)pyridine 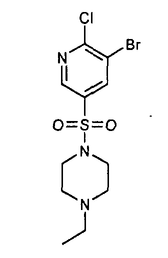 A solution of 1-ethylpiperazine (11.3ml, 89.0mmol) and triethylamine (12.5ml, 89.0mmol) in dichloromethane (150ml) was added dropwise to an ice-cooled solution of the product from stage c) (23.0g, 79.0mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the title compound as an orange solid, 14.5g; δ (CDCl₃, 300MHz): 1.05 (3H, t), 2.42 (2H, q), 2.55 (4H, m), 3.12 (4H, m), 8.24 (1H, s), 8.67 (1H, s).
e) 3-Bromo-2-ethoxy-5-(4-ethylpiperazin-1-ylsulphonyl)pyridine 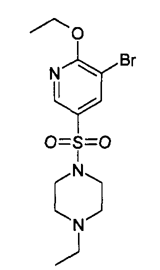 A mixture of the product from stage d) (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the title compound as a brown solid, 6.41g; Found : C, 41.27; H, 5.33; N, 11.11. C₁₃H₂₀BrN₃O₃S requires C, 41.35; H, 5.28; N, 10.99%; δ (CDCl₃, 300MHz): 1.06 (3H, t), 1.48 (3H, t), 2.42 (2H, q), 2.56 (4H, m), 3.09 (4H, m), 4.54 (2H, q), 8.10 (1H, s), 8.46 (1H, s); LRMS : m/z 378, 380 (M+1)⁺.
f) Pyridine 2-ethoxy-5-(4-ethylpiperazin-1-ylsulphonyl)-3-carboxylic acid ethyl ester 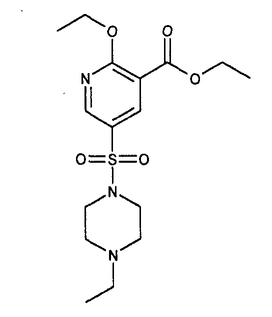 A mixture of the product from stage e) (6.40g, 16.92mmol), triethylamine (12ml, 86.1 mmol), and palladium (0) tris(triphenylphosphine) in ethanol (60ml) was heated at 100°C and 200 psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the title compound as an orange oil, 6.2g; δ (CDCl₃, 300MHz) : 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s); LRMS : m/z 372 (M+1)⁺
g) Pyridine 2-ethoxy-5-(4-ethylpiperazin-1-ylsulphonyl)-3-carboxylic acid 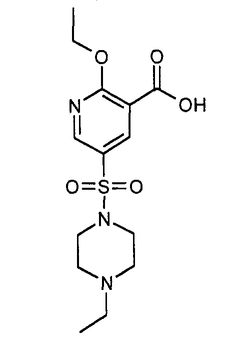 A mixture of the product from stage f) (4.96g, 13.35mmol) and aqueous sodium hydroxide solution (25ml, 2N, 50.0mmol) in ethanol (25ml) was stirred at room temperature for 2 hours. The reaction mixture was concentrated under reduced pressure to half it's volume, washed with ether and acidified to pH 5 using 4N hydrochloric acid. The aqueous solution was extracted with dichloromethane (3x30ml), the combined organic extracts dried (MgSO₄) and evaporated under reduced pressure to afford the title compound as a tan coloured solid, 4.02g; δ (DMSOd₆, 300MHz): 1.18 (3H, t), 1.37 (3H, t), 3.08 (2H, q), 3.17-3.35 (8H, m), 4.52 (2H, q), 8.30 (1H, s), 8.70 (1H, s).
h) 4-[2-Ethoxy-5-(4-ethylpiperazin-1-ylsulphonyl)pyridin-3-ylcarboxamidol-1H-3- ethylpyrazole-5-carboxamide 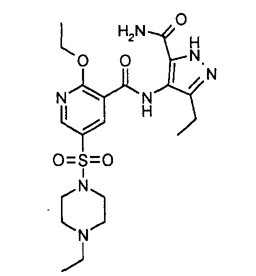 A solution of 3-ethyl-1H-pyrazole-5-carboxamide (WO 9849166) (9.2g, 59.8mmol) in N,N-dimethylformamide (60ml) was added to a solution of the product from stage g) (21.7g, 62.9mmol), 1-hydroxybenzotriazole hydrate (10.19, 66.0mmol) and triethylamine (13.15ml, 94.3mmol) in dichloromethane (240ml). 1-(3-Dimethylaminopropyl)-3-ethylcarbodiimide hydrochloride (13.26g, 69.2mmol) was added and the reaction stirred at room temperature for 6 hours. The dichloromethane was removed under reduced pressure, the remaining solution poured into ethyl acetate (400ml), and this mixture washed with aqueous sodium bicarbonate solution (400ml). The resulting crystalline precipitate was filtered, washed with ethyl acetate and dried under vacuum, to afford the title compound, as a white powder, 22g; δ (CDCl₃+1 drop DMSOd₆) 0.96 (3H, t), 1.18 (3H, t), 1.50 (3H, t), 2.25-2.56 (6H, m), 2.84 (2H, q), 3.00 (4H, m), 4.70 (2H, q), 5.60 (1 H, br s), 6.78 (1 H, br s), 8.56 (1 H, d), 8.76 (1 H, d), 10.59 (1H, s), 12.10-12.30 (1H, s); LRMS: m/z 480 (M+1)⁺.
i) 2-Methoxyethyl-4-[2-ethoxy-5-(4-ethylpiperazin-1-ylsulphonyl)pyridin-3-ylcarboxamido]-3-ethylpyrazole-5-carboxamide 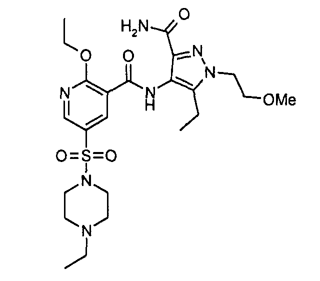 1-Bromo-2-methoxyethane (1.72mmol) was added to a solution of the product from stage h) (750mg, 1.56mmol) and caesium carbonate (1.12g, 3.44mmol) in N,N-dimethylformamide (15ml) and the reaction stirred at 60°C for 18 hours. The cooled mixture was partitioned between water and ethyl acetate, and the layers separated. The organic layer was dried (MgSO₄), concentrated under reduced pressure and azeotroped with toluene to give a solid. This product was recrystallised from ether, to afford the title compound as a white solid.

### Example 2

### 5-[2-iso-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one

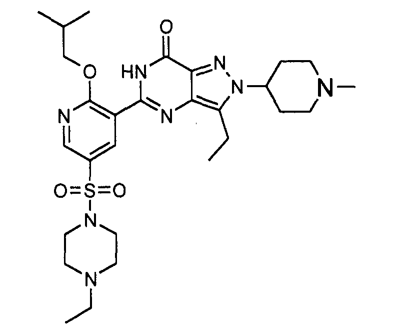

A mixture of the product from stage b) below (90mg, 0.156mmol), potassium bis(trimethylsilyl)amide (156mg, 0.78mmol) and ethyl acetate (14mg, 0.156mmol) in iso-propanol (12ml) was stirred at 130°C for 6 hours in a sealed vessel. The cooled reaction mixture was poured into saturated aqueous sodium bicarbonate solution (60ml), and extracted with ethyl acetate (60ml). The combined organic extracts were dried (MgSO₄), and evaporated under reduced pressure to give a gum. The crude product was purified by column chromatography on silica gel using dichloromethane:methanol:0.88 ammonia (92.6:6.6:0.6) to afford the title compound as a beige foam, 36 mg; δ (CDCl₃) 1.01 (3H, t), 1.12 (6H, d), 1.39 (3H, t), 1.94 (2H, m), 2.15 (2H, m), 2.22-2.44 (6H, m), 2.55 (6H, m), 3.02 (4H, m), 3.14 (4H, m), 4.22 (1H, m), 4.43 (2H, d), 8.60 (1H, d), 9.00 (1H, d), 10.54 (1H, s).

### Preparation of Starting Materials

a) 2-(1-*tert*-Butoxycarbonylpiperidin-4-yl)-4-[2-ethoxy-5-(4-ethylpiperazin-1-ylsulphonyl)pyridin-3-ylcarboxamido]-3-ethylpyrazole -5-carboxamide 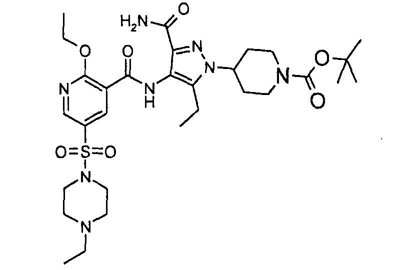 Sodium hydride (64mg, 60% dispersion in mineral oil, 1.6mmol) was added to a solution of the product from Example 1, stage h) (1.46mmol) in tetrahydrofuran (10ml), and the solution stirred for 10 minutes. *tert*-Butyl 4-[(methylsulphonyl)oxy]-1-piperidinecarboxylate (WO 9319059) (1.60mmol) was added and the reaction stirred at 60°C for 3 days. The cooled mixture was partitioned between ethyl acetate and aqueous sodium bicarbonate solution, and the phases separated. The aqueous layer was extracted with ethyl acetate, the combined organic solutions dried (MgSO₄) and evaporated under reduced pressure. The residue was purified by column chromatography on silica gel using dichloromethane:methanol (98:2) as eluant to afford the title compound as a white foam, 310 mg; δ (CDCl₃) 1.02 (3H, t), 1.23 (3H, t), 1.49 (9H, s), 1.57 (3H, m), 1.93 (2H, m), 2.16 (2H, m), 2.40 (2H, q), 2.54 (4H, m), 2.82-2.97 (4H, m), 3.10 (4H, m), 4.30 (3H, m), 4.79 (2H, q), 5.23 (1H, s), 6.65 (1H, s), 8.63 (1H, d), 8.82 (1H, d), 10.57 (1H, s).
b) 4-[2-Ethoxy-5-(4-ethylpiperazin-1-ylsulphonyl)pyridin-3-ylcarboxamido]-3-ethyl-2-(1-methylpiperidin-4-yl)pyrazole-5-carboxamide 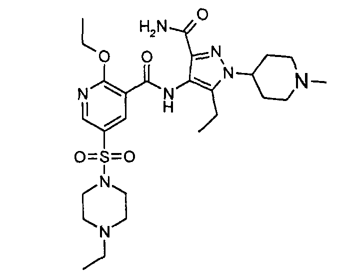 Trifluoroacetic acid (1.5ml) was added to a solution of the product from stage a) above (320mg, 0.48mmol) in dichloromethane (2ml) and the solution stirred at room temperature for 2 ½ hours. The reaction mixture was evaporated under reduced pressure and the residue triturated well with ether and dried under vacuum, to provide a white solid. Formaldehyde (217 microlitres, 37% aqueous, 2.90mmol) was added to a solution of the intermediate amine in dichloromethane (8ml), and the solution stirred vigorously for 30 minutes. Acetic acid (88 microlitres, 1.69mmol) was added, the solution stirred for a further 30 minutes, then sodium triacetoxyborohydride (169mg, 0.80mmol) was added and the reaction stirred at room temperature for 16 hours. The reaction mixture was poured into aqueous sodium bicarbonate solution, and extracted with ethyl acetate. The combined organic extracts were dried (MgSO₄) and evaporated under reduced pressure. The residue was purified by column chromatography on silica gel using dichloromethane:methanol:0.88 ammonia (91.75:7.5:0.75) as eluant to afford the title compound, 70mg; δ (CDCl₃) 1.02 (3H, t), 1.22 (3H, t), 1.58 (3H, t), 1.92 (2H, m), 2.14 (2H, m), 2.25-2.45 (7H, m), 2.54 (4H, m), 2.91 (2H, q), 2.99-3.16 (6H, m), 4.08 (1H, m), 4.78 (2H, q), 5.11 (1H, br s), 6.65 (1H, br s), 8.63 (1H, d), 8.83 (1H, d), 10.53 (1H, s).

### Example 3

### 5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one

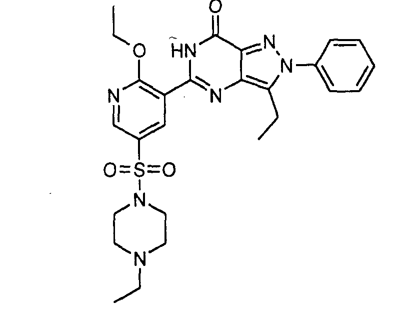

Pyridine (0.1 ml, 1.08mmol) was added to a mixture of the product from stage a) below (250mg, 0.54mmol), copper (II) acetate monohydrate (145mg, 0.72mmol), benzeneboronic acid (132mg, 1.08mmol) and 4Å molecular sieves (392mg) in dichloromethane (5ml), and the reaction stirred at room temperature for 4 days. The reaction mixture was filtered and the filtrate evaporated under reduced pressure. The crude product was purified by column chromatography on silica gel using dichloromethane:methanol:0.88 ammonia (97:3:0.5) as eluant, and triturated with ether:hexane. The resulting solid was filtered and recrystallised from *iso*propanol:dichloromethane to give the title compound as a solid, 200mg, δ (CDCl₃) 1.02 (3H, t), 1.47 (3H, t), 1.60 (3H, t), 2.42 (2H, q), 2.58 (4H, m), 3.10 (2H, q), 3.17 (4H, m), 4.76 (2H, q), 7.40 (1H, m), 7.51 (2H, m), 7.80 (2H, d), 8.67 (1H, d), 9.16 (1H, s), 10.90 (1H, s); LRMS : m/z 538 (M+1)⁺.

### Preparation of Starting Materials

a) 5-[2-Ethoxy-5-(4-ethylpiperazin-1-ylsulphonyl)pyridin-3-yl]-3-ethyl-2,6-dihydro-7H-pyrazolo[4,3-d]pyrimidin-7-one 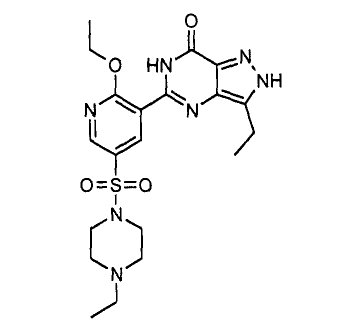 Potassium bis(trimethylsilyl)amide (8.28g, 41.6mmol) was added to a solution of the product from Example 1, stage h) (10.0g, 20.8mmol) and ethyl acetate (2ml, 20mmol) in ethanol (160ml), and the reaction mixture heated at 120°C for 12 hours in a sealed vessel. The cooled mixture was evaporated under reduced pressure and the residue was purified by column chromatography on silica gel using dichloromethane:methanol:0.88 ammonia (95:5:0.5) as eluant, to give the title compound, 3.75g; δ (CDCl₃) 1.03 (3H, t), 1.42 (3H, t), 1.60 (3H, t), 2.42 (2H, q), 2.58 (4H, m), 3.02 (2H, q), 3.16 (4H, m), 4.78 (2H, q), 8.66 (1H, d), 9.08 (1H, d), 11.00 (1H, s) 11.05-11.20 (1H, br s), LRMS : m/z 462 (M+1)⁺.

### Example 4

### 5-(5-Acetyl-2-propoxy-3-pyridinyl)-3-ethyl-2-(1-isopropyl-3-azetidinyl)-2,6-dihydro-7H-pyrazolo[4,3-d]pyrimidin-7-one

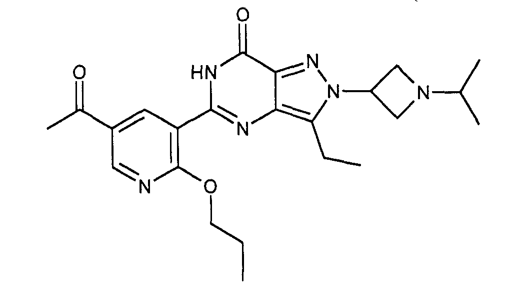

The product from stage h) below (0.23 mmol) was dissolved in dichloromethane (10 ml) and acetone (0.01 ml) was added. After 30 min stirring sodium triacetoxyborohydride (0.51 mmol) was added and stirring continued for 14 h. Further acetone (0.01 ml) and sodium triacetoxyborohydride (0.51 mmol) were added and stirring continued for a further 4.5 h. Starting material still remained so further acetone (0.01 ml) and sodium triacetoxyborohydride (0.51 mmol) were added and stirring continued for a further 18 h. The reaction mixture was diluted with dichloromethane, washed with sodium bicarbonate solution then brine, dried (MgSO₄) and concentrated. Purification by flash column chromatography (elution with 94:6:0.6 dichloromethane/methanol/0.88 ammonia) gave the product as a solid, M.p. 162.8-163.6°C; 1H NMR (400MHz, MeOD): δ = 1.00 (app. d, 9H), 1.30 (t, 3H), 1.84 (app. q, 2H), 2.60 (s, 3H), 2.62-2.72 (m, 1H), 3.00-3.10 (q, 2H), 3.75 (t, 2H), 3.90 (t, 2H), 4.50 (t, 2H), 5.25 (t, 1H), 8.70 (s, 1H), 8.90 (s, 1H); LRMS (TSP - positive ion) 439 (MH⁺); Anal. Found C, 61.92; H, 6.84; N, 18.70 Calcd for C₂₃H₃₀O₃N₆.0.1CH₂Cl₂: C, 62.07; H, 6.81; N, 18.80.

### Preparation of Starting Materials

a) 2-Propoxy-5-iodonicotinic acid 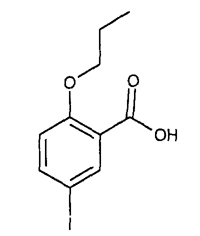 *N*-lodosuccinamide (18.22 g, 0.08 mol), trifluoroacetic acid (100 ml) and trifluoroacetic anhydride (25 ml) were added to 2-propoxynicotinic acid (0.054 mol). The mixture was refluxed for 2.5 h, cooled and the solvents evaporated. The residue was extracted from water with ethyl acetate and the organics washed with water (twice) and brine (twice), dried (MgSO₄) and concentrated. The red residue was redissolved in ethyl acetate washed with sodium thiosulfate solution (twice), water (twice), brine (twice), redried (MgSO₄) and concentrated to give the desired product as a solid; ¹H NMR (300 MHz, CDCl₃): δ = 1.05 (t, 3H), 1.85-2.0 (m, 2H), 4.5 (t, 2H), 8.5 (s, 1H), 8.6 (s, 1H); Analysis: found C, 35.16; H, 3.19; N, 4.46. Calcd for C₉H₁₀INO₃: C, 35.19; H, 3.28; N, 4.56%; LRMS (TSP): 529.5 (MH⁺).
b) *N*-[3-(Aminocarbonyl)-5-ethyl-1*H*-pyrazol-4-yl]-5-iodo-2-propoxy-nicotinamide 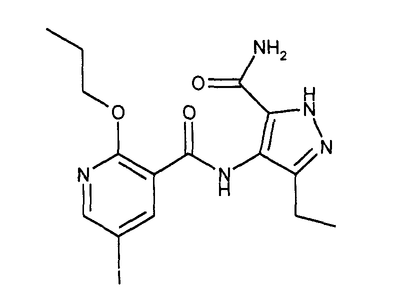 Oxalyl chloride (15.9 mmol) was added to a stirred solution of the product from stage a) (3.98 mmol) in dichloromethane (20 ml) and 3 drops *N,N*-dimethylformamide added. After 2.5 h the solvent was evaporated and the residue azeotroped 3 times with dichloromethane. The residue was resuspended in dichloromethane (4 ml) and added to a stirred mixture 4-amino-3-ethyl-1*H*-pyrazole-5-carboxamide (prepared as described in WO 98/49166) (3.58 mmol) and triethylamine (7.97 mmol) in dichloromethane (10 ml). After 1 h the solvent was evaporated and the residue partitioned between ethyl acetate and water. The organic phase was separated and washed with 2N HCI (twice), sodium bicarbonate solution (twice) and brine before being dried (MgSO₄) and concentrated. The product was triturated with ether and filtered to give the title product as a solid. The mother liquor was concentrated and purified by flash column chromatography (elution with 80% ethyl acetate : hexane) to give further product; ¹H NMR (300 MHz, d₄-MeOH): δ = 1.0 (t, 3H), 1.25 (t, 3H), 1.85-2.0 (m, 2H), 2.8 (q, 2H), 4.5 (t, 2H), 8.5 (s, 1H), 8.6 (s, 1H); LRMS (TSP) 444 (MH⁺).
c) *tert*-Butyl 3-iodo-1-azetidinecarboxylate 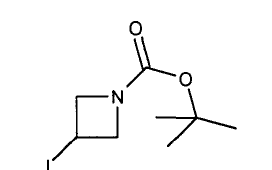 A mixture of *tert*-butyl 3-[(methylsulfonyl)oxy]-1-azetidinecarboxylate (prepared as described in *Synlett* 1998, 379; 5.0 g, 19.9 mmol), and potassium iodide (16.5 g, 99.4 mmol) in N,N-dimethylformamide (25 ml), was heated at 100°C for 42 h. The cooled mixture was partitioned between water and ethyl acetate, and the layers separated. The organic phase was dried over MgSO₄, concentrated under reduced pressure and the residue azeotroped with xylene. The crude product was purified by flash column chromatography (dichloromethane as eluant) to give the title compound, 3.26 g; ¹H NMR (300 MHz, CDCl₃) δ = 1.43 (s, 9H), 4.28 (m, 2H), 4.46 (m, 1H), 4.62 (m, 2H); LRMS (TSP) 284 (MH)⁺
d) *tert*-Butyl 3-(3-(aminocarbonyl)-5-ethyl-4-{[(5-iodo-2-propoxy-3-pyridinyl)carbonyl]amino}-1*H*-pyrazol-1-yl)-1-azetidinecarboxylate 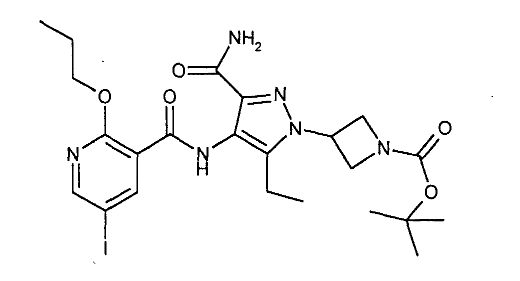 Cesium carbonate (3.59 mmol) was added to a stirred solution of the product from stage b) (1.79 mmol) and the product from stage c) (2.15 mmol) in *N,N*-dimethylformamide (10 ml) under a nitrogen atmosphere. The mixture was heated at 80°C for 24 h. The mixture was cooled and extracted from water with ethyl acetate. The organics were dried (MgSO₄) and concentrated to give a brown oil. Purification by flash column chromatography (gradient elution from 100% dichloromethane to 90% dichloromethane/MeOH) gave the title product; 1H NMR (400MHz, DMSO): δ = 0.95 (t, 3H), 1.05 (t, 3H), 1.40 (s, 9H), 1.78-1.88 (m, 2H), 2.68 (q, 2H), 4.22-4.35 (m, 4H), 4.40 (t, 2H), 5.33 (t, 1H), 7.35 (bs, 1H), 7.52 (bs, 1H), 840 (s, 1H), 8.55 (s, 1H), 10.10 (s, 1H); LRMS (TSP - positive ion) 373.2 (MH⁺ - BOC and I); Anal. Found C, 45.11; H, 5.07; N, 13.56 Calcd for C₂₃H₃₁O₅N₆I. 0.2 DCM: C, 45.28; H, 5.14; N, 13.66.
e) *tert*-Butyl 3-[3-ethyl-5-(5-iodo-2-propoxy-3-pyridinyl)-7-oxo-6,7-dihydro-2H-pyrazolo[4,3-*d*]pyrimidin-2-yl]-1-azetidinecarboxylate 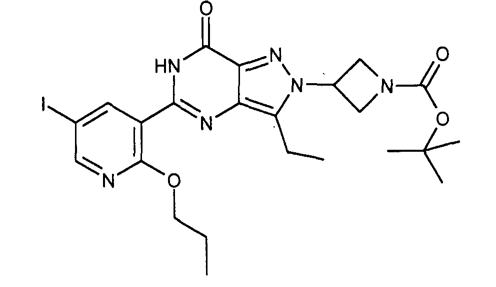 The product from stage d) (28.4 mmol) was dissolved in n-propanol (200 ml), ethyl acetate (6 ml) and potassium t-butoxide (28.4 mmol) were added and the resultant mixture heated to reflux for 6h. Additional potassium t-butoxide (14.2 mmol) was added and the mixture heated for a further 2h, after which the solvent was removed *in vacuo.* The residue was partioned between water (50 ml) and methylene chloride (100 ml) and the organic phase separated. The aqueous phase was extracted with dichloromethane (2 x 100 ml) and the combined organics dried over MgSO₄ and reduced to a solid. Purification by column chromatography (elution with ethyl acetate) gave the title compound; 1H NMR (400MHz, CDCl₃): δ = 1.05 (t, 3H), 1.30 (t, 3H), 1.43 (s, 9H), 1.87-1.96 (m, 2H), 3.00 (q, 2H), 4.34 (t, 2H), 4.49 (t, 2H), 4.60 (br s, 2H), 5.20 (t, 1H), 8.41 (d, 1H), 8.94 (s, 1H), 10.75 (br s, 1H); LRMS (TSP - positive ion) 598.1 (MNH₄⁺); Anal. Found C, 47.54; H, 5.02; N, 14.09 Calcd for C₂₃H₂₉O₄N₆I: C, 47.60; H, 5.04; N, 14.48.
f) *tert*-Butyl 3-(3-ethyl-7-oxo-5-{2-propoxy-5-[(trimethylsilyl)ethynyl]-3-pyridinyl}-6,7-dihydro-2*H*-pyrazolo[4,3-*d*]pyrimidin-2-yl)-1-azetidinecarboxylate 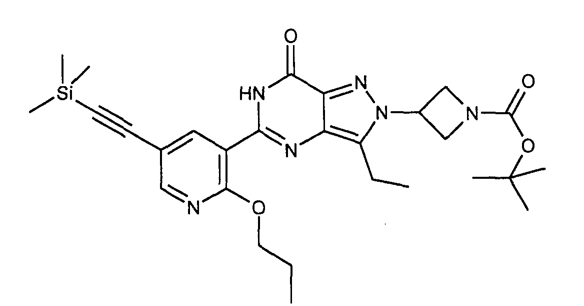 The product from stage e) (0.25 mmol) was suspended in triethylamine (2 ml) and trimethylsilylacetylene (0.39 mmol) and acetonitrile (2 ml to try and solubilise reactants). Pd(PPh₃)₂Cl₂ (0.006 mmol) and cuprous iodide (0.006 mmol) were added and the reaction mixture stirred. After 1 h a further portion of trimethylsilylacetylene (0.19 mmol) was added and stirring continued for 2 h. The solvent was evaporated and the residue partitioned between ethyl acetate and water. The organics were washed with brine, dried (MgSO₄) and concentrated. Purification by flash column chromatography (gradient elution from 100% dichloromethane to 99% dichloromethane/methanol) gave the title compound; 1H NMR (400MHz, MeOD): δ = 0.25 (s, 9H), 1.05 (t, 3H), 1.31 (t, 3H), 1.44 (s, 9H), 1.87-1.96 (m, 2H), 3.00 (q, 2H), 4.33 (t, 2H), 4.52 (t, 2H), 4.54-4.80 (m, 2H), 5.18-5.25 (m, 1H), 8.32 (d, 1H), 8.74 (d, 1H); LRMS (TSP-positive ion) 569 (MNH₄⁺), 452.0 (MH⁺); Anal. Found C, 60.82; H, 6.90; N, 15.15 Calcd for C₂₈H₃₈O₄N₆Si: C, 61.07; H, 6.95; N, 15.26.
g) *tert*-Butyl 3-[3-ethyl-5-(5-ethynyl-2-propoxy-3-pyridinyl)-7-oxo-6,7-dihydro-2H-pyrazolo[4,3-*d*]pyrimidin-2-yl]-1-azetidinecarboxylate 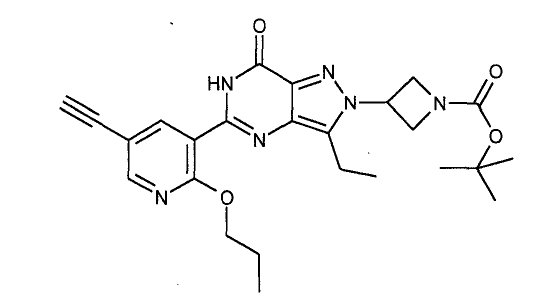 Potassium fluoride (0.38 mmol) was added to a stirred solution of the product of stage f) (0.19 mmol) in aqueous N,N-dimethylformamide (2 ml *N,N*-dimethylformamide /0.2 ml water) at 0°C. After 10 min the reaction was allowed to warm to room temperature and stirred for 2 h. The reaction mixture was diluted with ethyl acetate and washed with water, 1 *N* hydrochloric acid (3 times) and brine. The organic layer was dried (MgSO₄) and concentrated to give the title compound as a solid; 1H NMR (400MHz, CDCl₃): δ = 1.05 (t, 3H), 1.30 (t, 3H), 1.43 (s, 9H), 1.88-2.00 (m, 2H), 3.00 (q, 2H), 3.19 (s, 1H), 4.35 (app t, 2H), 4.52 (app t, 2H), 4.60-4.80 (br s, 2H), 5.22 (t, 1H), 8.39 (s, 1H), 8.80 (s, 1H), 10.75 (br s, 1H); LRMS (TSP - positive ion) 496 (MNH₄⁺).
h) 5-(5-Acetyl-2-propoxy-3-pyridinyl)-2-(3-azetidinyl)-3-ethyl-2,6-dihydro-7*H*- pyrazolo[4,3-*d*]pyrimidin-7-one 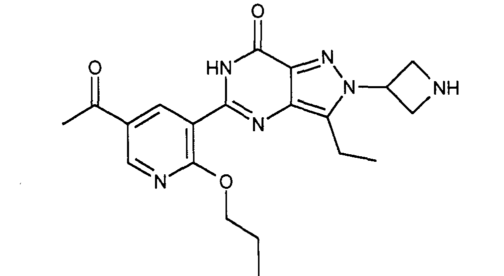 The product from stage g) (1.44 g, 3.0 mmol) in acetone (50 ml) and sulphuric acid (1N, 3 ml) was treated with mercuric sulphate (268 mg, 9.0 mmol) and heated to reflux for 6h. The reaction mixture was concentrated to ~20 ml *in vacuo,* poured into sodium bicarbonate (sat. aq., 20ml) and extracted into methylene chloride (6 x 20 ml). Combined organics were washed with brine (20 ml), dried over MgSO₄, and concentrated to a brown oil which was taken up in 40% trifluoroacetic acid in methylene chloride (50ml) and water (1 ml) and stirred for 1h at room temperature. After evaporation *in vacuo*, the residue was purified by column chromatography (eluting with 95:5:1 methylene chloride:methanol:0.88 ammonia) to afford the title compound as a white hydroscopic foam (1.65 g); m.p. 128.5-130.0°C; 1H NMR (400MHz, MeOD): δ = 1.00 (t, 3H), 1.30 (t, 3H), 1.79-1.90 (m, 2H), 2.60 (s, 3H), 3.00-3.10 (q, 2H), 4.50 (t, 2H), 4.60-4.70 (m, 4H), 5.65-5.78 (m, 1H), 8.65 (s, 1H), 8.90 (s, 1H); LRMS (TSP - positive ion) 397 (MH⁺).

### Example 5

### 5-(5-Acetyl-2-butoxy-3-pyridinyl)-3-ethyl-2-(1-ethyl-3-azetidinyl)-2,6-dihydro-7H-pyrazolo[4,3-d]pyrimidin-7-one

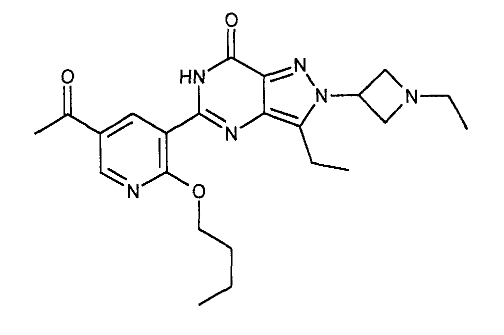

The starting material (120 mg, 0.28 mmol) and cesium carbonate (274 mg, 0.84 mmol) were dissolved in n-butanol (4 ml), and heated at 90°C under nitrogen with molecular sieves for 96h. The mixture was then partitioned between water (10 ml) and dichloromethane (10 ml). The organic layer was separated, and the aqueous layer extracted further with dichloromethane (3 x 15 ml). The combined organic layers were dried (MgSO₄), and concentrated *in vacuo*. The crude product was purified by flash column chromatography (95:5:0.5-90:10:1 ethyl acetate:methanol:0.88 NH₃ as eluents), to yield the title compound as a colourless glass (77 mg, 0.18 mmol); m.p. 91.6-93.7°C; 1 H NMR (400MHz, CDCl₃): δ = 1.00-1.05 (m, 6H), 1.38 (t, 3H), 1.50-1.62 (m, 2H), 1.90-2.00 (m, 2H), 2.63 (s, 3H), 2.63-2.70 (m, 2H), 3.02 (q, 2H), 3.75 (t, 2H), 3.90 (t, 2H), 4.68 (t, 2H), 5.10-5.20 (m, 1H), 8.84 (s, 1H), 9.23 (s, 1H), 10.63 (br s, 1H); LRMS (TSP - positive ion) 439 (MH⁺);
Anal. Found C, 60.73; H, 7.06; N, 18.03 Calcd for C₂₃H₃₀O₃N₆.0.2MeOH.0.1 DIPE: C, 60.88; H, 7.26; N, 17.90.

### Preparation of Starting Materials

### 5-(5-Acetyl-2-propoxy-3-pyridinyl)-3-ethyl-2-(1-ethyl-3-azetidinyl)-2,6-dihydro-7H-pyrazolo[4,3-d]pyrimidin-7-one

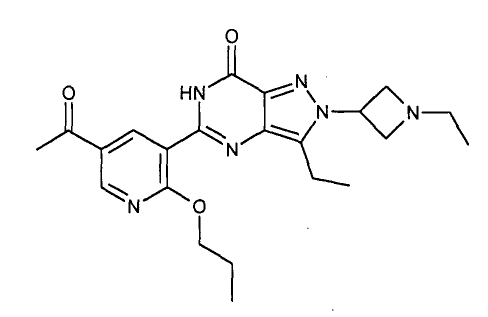

Sodium cyanoborohydride (92 mg, 1.47 mmol) was added to a stirred solution of the product from Example 4 stage h) (500 mg, 0.98 mmol) and sodium acetate (161 mg, 1.96 mmol) in methanol (10 ml) under nitrogen at room temperature. After 1h the mixture was poured into NaHCO₃ (sat. aq., 20 ml), and extracted with dichloromethane (3 x 15 ml). The combined organic layers were dried (MgSO₄) and concentrated *in vacuo.* The crude product was purified by flash column chromatography (95:5:0.5-80:20:1 ethyl acetate:methanol:0.88 NH₃ as eluent) to yield the title compound as a white solid (140 mg, 0.33 mmol); 1H NMR (400MHz, CDCl₃): δ = 0.97 (t, 3H), 1.03 (t, 3H), 1.30 (t, 3H), 2.82-2.97 (m, 2H), 2.58-2.65 (m, 5H), 2.98 (q, 2H), 3.68 (t, 2H), 3.85 (dd, 2H), 4.58 (dd, 2H), 5.05-5.17 (m, 1H), 8.79 (s, 1H), 9.18 (s, 1H), 10.62 (br s, 1H); LRMS (TSP - positive ion) 426 (MH⁺).

## Claims

1. A method of treating a patient suffering from neuropathy which comprises treating said patient with an effective amount of a cGMP PDE5 inhibitor, with the proviso that the inhibitor is not a:
i) substituted 5-(3-pyridyl)pyrazolo[4,3-d]pyrimidin-7-one,
ii) substituted 2-(3-pyridyl)-4a,5-dihydroimidazo[5,1-*f*][1,2,4]triazin-4(3*H*)-one, or
iii) substituted 2-phenylpurin-6-one or a substituted 2-(3-pyridyl)purin-6-one,
for treating peripheral diabetic neuropathy.

2. A method according to claim 1 wherein the neuropathy is diabetic polyneuropathy.

3. A method according to claim 1 or 2 wherein the inhibitor is administered orally

4. A method according to claim 3 wherein the daily dosage is 5 to 500 mg.

5. A method according to any preceding claim wherein the inhibitor has an IC50 at less than 100 nanomolar.

6. A method according to any preceding claim wherein the inhibitor has a selectivity ratio in excess of 1000.

7. A method according to any preceding claim wherein the inhibitor is sildenafil, or pharmaceutically acceptable salts thereof.

8. A method according to claim 7 wherein the daily dosage is 10 to 100 mg.

9. The use of a cGMP-PDE5 inhibitor for the manufacture of a medicament for the treatment of neuropathy, with the proviso that the inhibitor is not a:
i) substituted 5-(3-pyridyl)pyrazolo[4,3-d]pyrimidin-7-one,
ii) substituted 2-(3-pyridyl)-4a,5-dihydroimidazo[5,1-*f*][1,2,4]triazin-4(3*H*)-one, or
iii) substituted 2-phenylpurin-6-one or a substituted 2-(3-pyridyl)purin-6-one,
for treating peripheral diabetic neuropathy.

10. The use according to claim 9 wherein the neuropathy is diabetic polyneuropathy.

11. The use according to claim 9 or 10 wherein the inhibitor is administered orally.

12. The use according to claim 11 wherein the daily dosage is 5 to 500 mg.

13. The use according to any one of claims 9 to 12 wherein the inhibitor has an IC50 at less than 100 nanomolar.

14. The use according to any one of claims 9 to 13 wherein the inhibitor has a selectivity ratio in excess of 1000.

15. The use according to any one of claims 9 to 14 wherein the inhibitor is sildenafil, or pharmaceutically acceptable salts thereof.

16. The use according to claim 15 wherein the daily dosage is 10 to 100 mg.
